# EUROPÄISCHE PATENTSCHRIFT

(11) **EP 0 447 672 B1**
(45) Veröffentlichungstag und Bekanntmachung des Hinweises auf die Patenterteilung: **08.03.1995**
(21) Anmeldenummer: 90125379.9
(22) Anmeldetag: 22.12.1990
(51) Int. Cl.: D21C 5/02, D21C 5/00

(54) **Verfahren zur Altpapieraufbereitung mit enzymatischer Druckfarbenentfernung**
Process of treating waste paper by enzymatic de-inking
Procédé pour le traitement de vieux papier par désencrage enzymatique

(30) Priorität: 20.03.1990 DE 4008894
(43) Veröffentlichungstag der Anmeldung: 25.09.1991
(73) Patentinhaber: LIGNOZYM GESELLSCHAFT ZUR HERSTELLUNG UND ZUM VERTRIEB VON ENZYMEN mbH, D-52499 Baesweiler (DE)
(72) Erfinder: Call, Hans-Peter Dr., D-52531 Übach-Palenberg (DE)
(74) Vertreter: Fitzner, Ulrich, Dr.

(56) Entgegenhaltungen:
- WO-A-88/03190
- ABSTRACT BULLETIN OF THE INSTITUTE OF PAPER CHEMISTRY. vol. 59, no. 3,September 1988, APPLETON US Seite 373 Nomura,Y.; Shoji,S.: "Elimination of inkfrom reclaimed paper."
- PAPIER, DAS. vol. 44, no. 10A, Oktober 1990, DARMSTADT DE Seiten V33 - V41;Call,H.P. et al.: "Einsatz von lignolytischen Enzymen bei der Zellstoff- undPapierherstellung (Biopulping, Biobleaching)."

## Beschreibung

Die vorliegende Erfindung betrifft ein Verfahren zur Altpapieraufbereitung mit enzymatischer Druckfarbenentfernung.

In der betrieblichen Praxis ist die Altpapieraufbereitung mit Druckfarbenentfernung (Deinking) eine Verkettung verschiedener Teilprozesse. Dabei haben die Auswahl der einzusetzenden Aggregate und Auslegung des Gesamtsystems immer in Bezug auf das Endprodukt, unter Berücksichtigung des wirtschaftlichen Aspekts der Kostenminimierung und des technologischen Aspekts der Optimierung der Qualitätseigenschaften der Halbstoffe, zu erfolgen. Das Ziel einer Altpapieraufbereitung im Deinking-Prozeß ist die möglichst quantitative Entfernung von Druckfarbenpartikeln bei minimalem Verlust jener Stoffe, die für die anschließende Papierherstellung wertvoll sind. Unmittelbar mit diesem Ziel ist der Wunsch verbunden, den heterogenen Altpapier-Eingangsstoff von all den festen, gelösten oder kolloidalgelösten Substanzen so zu befreien, daß die Papierherstellung nicht störend beeinflußt und die Qualität des Papiers nicht gemindert wird. Welche Altpapierinhaltsstoffe für die Altpapierherstellung wertvoll sind, richtet sich nach den Qualitätsanforderungen des Endprodukts. So sind z.B. Füllstoffe bei Zeitungsdruckpapier in gewissen Grenzen ein wertvoller Bestandteil, bei der Herstellung von Tissue-Papieren dagegen unerwünscht. Demgemäß sind nach dem Stand der Technik eine Vielzahl von Verfahren untersucht und vorgeschlagen worden (Lausch, H.: Ortner, H.: "Über den Einfluß der Druckfarbenzusammensetzung auf den Deinkingprozeß" Wochenbl. f. Papierf. 94 (1966). Nr. 5. S. 129-136; Berndt. W.: "Druckfarben und Deinken" Wochenbl. f. Papierf. 104 (1976). Nr. 3, 95-98; Forester. W.K.: "Deinking of UV-cured links" Tappi J. (1987), Nr. 5, 127-130; N.N.: "Deinkbarkeit von Flexodruck-Tageszeitungen" IFRA Special Report 1.3. Darmstadt, Oktober 1987; Putz. H.J.: "Upcyling von Altpapier für den Einsatz in höherwertigen graphischen Papieren durch chemisch-mechänische Aufbereitung (Deinken und Bleichen)" Dissertation, Technische Hochschule Darmstadt, 1987; Weidhass. A.G.: "Erfahrungen mit einer Deinking Anlage zur Erzeugung von Zeitungsdruckpapier" Wochenbl. f. Papierf. 104 (1976), Nr. 22. 857-865; Lippert, G.V.: "Erfahrungen mit der Deinkinganlage für Zeitungsdruckpapier der Leykam-Mürztaler AG im Werk Bruck" Wochenbl. f. Papierf. 111 (1983), Nr. 15. 540-542). Alle diese Verfahren arbeiten mit physikalischchemischen Methoden.

Ein heute sehr weit verbreitetes Verfahren ist dies Flotationstechnik. Anfang der 60-iger Jahre wurde eine rechteckige Flotationszeile mit mechanische Verteilung der Flotationsluft von Voit entwickelt. Die Suspension gelangt in dieser Zelle zu einem Laufrad. das von einem konzentrisch angeordneten Lochmantel umgeben ist, sich am Boden der Zelle befindet und von oben angetrieben wird. Die Zelle arbeitet selbstansaugend. Das Laufrad sorgt für die Feindispergierung der Luft in der Suspension, für die Druckfarbenpartikel-Farben-Stöße und für die Verteilung des Stoffe über dem Boden der Zelle. Der Gutstoff wird über einen Überlauf abgeführt, der Schaum mit Hilfe eines Paddels ausgetragen.

Um die Hydrodynamik der Zelle zu verbessern wurde 1978 die Flotations-Rohr-Zelle entwickelt. Art der Belüftung sowie mechanische Feindispergierung der Luft wurden bei diesem Konzept von der geschilderten Flotationszelle übernommen.

Mit dem Ziel der Energiereduzierung, Verbesserung des Deinking-Wirkungsgrades und erhöhter Sicherheit wurde die Flotations-Rohr-Zelle 1982 neu konstruiert. Erhalten blieb ausschließlich die Form der Rohrzelle. Bei der Belüftung ging man von der mechanischen auf die Injektorbelüftung über. Der Injektor arbeitet nach dem Venturi-Prinzip selbstansaugend und erlaubt eine Belüftung bis zu 30 %. Der Schaumaustrag erfolgt selbsttätig ohne Paddel.

Im Jahre 1984 wurde die Kompakt-Flotationszelle vorgestellt. Für die Flotation in dieser Zelle sind zwei Faktoren von ausschlaggebender Bedeutung: 1) ein geeignetes Belüftungselement vor der Zelle: der Stufendifusor und 2) eine den Trennvorgang begünstigende Hydrodynamik der Zelle. Der belüftete Stoff wird an vier Stellen tangential der runden, stehenden Zelle zugeführt. Im Gegensatz zu allen anderen Flotationszellen erfolgt der Stoffzulauf nur unterhalb der Suspensionsoberfläche. Optimale Durchmischung von Luft und Suspension vorausgesetzt, soll eine sofortige gegenläufige Strömung in der Zelle (Luft mit Druckfarbenpartikeln nach oben und Gutstoff nach unten) das Wiederablösen der Druckfarbenpartikel von den Luftblasen vermeiden.

Weiterhin ist eine sogenannte Verticel-Flotationszelle bekannt, welche aus zwei übereinanderstehenden zylindrischen Behältern unterschiedlichen Durchmessers besteht. Der Stoff wird hierbei über Injektoren tangential in den unteren Behälter geleitet. Der Stoff-Luft-Suspensionsstrom steigt auf und gelangt über eine Trennwand in den oberen zylindrischen Behälter, der einen größeren Durchmesser hat. Beim Übersteigen der Trennwand werden die Luftblasen von der Schaumabsaugung erfaßt. Im äußeren Behälter wird der Stoff von unten tangential abgeführt und einem Niveaugefäß zugeleitet. Das Niveaugefäß sorgt für einen konstanten Abstand des Suspensionsspiegels vom Schaumabsaugrohr, so daß keine Niveauregelung notwendig ist.

Neben den vorgestellten Flotationsverfahren gibt es noch weitere Verfahren, welche hier nicht näher erläutert werden sollen. Gemeinsam ist allen diesen Verfahren, daß sie unter Einsatz von umweltbelastenden Chemikalien arbeiten.

Aus den US-A-4 687 745, 4 692 413 und 4 690 895 ist bekannt, daß sich die durch spezielle Mutanten des Pilzes Phanerochaete chrysosporium erzeugten Enzyme für die Entfärbung von zellstoffhaltigem Material eignen, was aber mit einem Deinking-Prozeß-nichts zu tun hat. Auch ist ein solches Verfahren zeitintensiv (ca. 12 Stunden). Zudem können die Enzyme nur aus dem speziell gezüchteten Stamm von Phanerochaete chrysosporium gewonnen werden.

Aus der WO-A-88/03190 ist ein Verfahren und ein Gerät zur Entfernung und/oder Umwandlung von Lignin oder dessen Abbauprodukten aus lignocellulosehaltigem Material bekannt. Hierbei wird ein durch Zusatz von Oxidations- und/oder Reduktionsmitteln und/oder Salzen und/oder phenolischen Verbindungen zu einer sauren wässrigen Lösung der ligninhaltigen Rohstoffe ein Redoxpotential im Bereich von 200 bis 500 mV eingestellt. Durch Zugabe on Enzymen, Mikroorganismen, tierischen oder pflanzlichen Zellen wird die ligninabbauende Reaktion mit gleichzeitiger Bleichung gestartet. Die Reaktion wird bei einem un einen konstanten Redoxpotential schwingenden Wert. konstanter Temperatur und ständigem Rühren über mehrere Stunden aufrechterhalten. Dieses Verfahren ist jedoch zur Papieraufbereitung mit Druckfarbenentfernung nicht geeignet. Denn der dort beschriebene Prozeß dient allein dem Abbau von Lignin aus Rohstoffen. z.B. aus Stroh.

Aus der JP-A-59494/88 ist schließlich ein Verfahren bekannt. bei dem Alkaliverbindungen, Wasserstoffperoxide, grenzflächenaktive Substanzen und Alkaliresistenten und Cellulasen der wässrigen Papiersuspension zugesetzt werden. Durch Flotation wird sodann die Druckfarbenentfernung erreicht. Nachteilig ist hier aber die Verfahrensdauer. Allein die enzymatische Reaktion dauert nämlich 1 bis 6 Stunden.

Die vorliegende Erfindung hat sich nunmehr die Aufgabe gestellt. ein Verfahren zur Altpapieraufbereitung mit enzymatischer Druckfarbenentfernung durch Flotation zur Verfügung zu stellen. welches die oben angeführten Nachteile der chemischen und enzymatischen Verfahren nicht aufweist.

Diese Aufgabe wird dadurch gelöst, daß
a) unter gleichzeitiger Zudosierung von Oxidations- und Reduktionsmitteln zu einer Altpapier enthaltenden Lösung ein Redoxpotential im Bereich von 200 bis 500 mV eingestellt,
b) durch Zugabe von lignotytischen Enzymen eine ligninabbauende Reaktion gestartet und
c) die Reaktion unter Zugabe von 60 bis 80 l/Std. Luft pro 1,5 l Reaktionsvolumen bei 40°C, o,8 bis 2,5 % Stoffdichte in einer handelsüblichen Flotationsanlage für ca. 10 Minuten fortgeführt wird.

Neu ist die erfindungsgemäße Kombination eines Enzymsystems mit den an sich bekannten Flotationsverfahren. Dadurch wird erreicht. daß anstelle der üblichen, oben geschilderten Flotationschemikalien, wie NaOH, H₂O oder Ölsäure die Druckfarbenentfernung weitgehend durch lignolytische Rohenzyme bewerkstelligt wird. Der eigentliche Prozeß des Entfärbens beruht bei dem erfindungsgemäßen Verfahren wahrscheinlich auf einer Lockerung der Fasern - wie bereits oben erwähnt - durch den Angriff des Enzyms auf das Lignin. Die Wirkung ist ähnlich der Quellwirkung von Natronlauge auf die Faser bei den konventionellen, eingangs erwähnten Entfärbungsverfahren. Es kommt also durch die Lockerung mittels der Enzyme zu einer Loslösung der Druckfarbenteilchen von der Faser. Aufgrund der aufgelockerten Struktur läßt sich im anschließenden Flotationsverfahren die Druckfarbe leicht entfernen, so daß meist nur eine zusätzliche Zudosierung eines Schäumers in geringsten Konzentrationnen nötig ist.

Zusätzlich können als Redoxmeditatoren Kupfer(II)sulfat, Mn(II)sulfat, Mn(II)acetat, Fe(II)sulfat, Ti(III)chlorid, Ce(IV)amoniumnitrat und Ce(III)nitrat verwendet werden. Ebenso eignen sich Zink-, Antimon- und Bleisalze.

Phenolische Verbindungen werden als Enzymschutzstoffe der Reaktion zugesetzt. Als solche eignen sich besonders Veratrylalkohole.

Neben den genannten Chemikalien können noch weitere Stoffe der Reaktionslösung zugesetzt werden. Hierzu gehören Natriumhypochlorit, Polysaccharide wie Glukane und/oder Xanthane, Detergenzien, Tenside, Fettsäuren wie Ölsäuren, Hämverbindungen wie Hämoglobin, und Bleichreagenzien, z.B. Natriumperborat. Zum Nachbleichen des entfärbten Papiers können gängige Bleichmittel wie Natriumhypochlorit, Sauerstoff, Chlordioxid, Ozon, H₂O₂ und Natriumdithionit eingesetzt werden.

Als Enzyme werden lignolytische Enzyme, wie Phenoloxidasen, Laccasen und Ligninperoxidasen eingesetzt. Hierzu zählen auch die aus dem Pilz Phanerochaete chrysosporium gewonnenen lignolytischen Enzyme. Es muß sich aber dabei nicht um die gemäß den erwähnten US-Patentschriften gewonnenen speziellen Enzymen handeln. Es ist erfindungsgemäß weder notwendig, die Enzyme in hochgereinigter Form noch die mittels spezieller optimierter Mutanten von Mikroorganismen gewonnenen Enzyme einzusetzen. Vielmehr reicht es aus, die heute handelsüblichen Rohenzyme für das erfindungsgemäße Verfahren einzusetzen. Zusätzlich zu den lignolytischen Enzymen können auch Pektinasen und/oder Hemicellulasen eingesetzt werden.

Der große Vorteil des erfindungsgemäßen Verfahrens beruht auf der nicht mehr notwendigen Vorbehandlung vor dem eigentlichen Flotationsprozeß mittels eingangs erwähnten Flotationschemikalien, welche nach dem Stand der Technik bis zu 90 Minuten dauerte. Dadurch, daß Laugen und ähnliche Stoffe nur noch in geringem Umfang dem Prozeß zugesetzt werden, zeichnet sich das erfindungsgemäße Verfahren durch seine umweltschonenden Abfallprodukte aus. Darüber hinaus ist es möglich, durch Vorschalten vor oder Nachschalten nach dem eigentlichen Flotationsprozeß eine Entfernung des Lignins aus dem Altpapier zu erreichen und hierdurch die mechanischen Eigenschaften zu verbessern. Z.B. kann die Reißlänge des behandelten Stoffes um mehr als 30 % bei einer Ligninentfernungsrate von ca. 5-8 % verbessert werden.

Daneben bleibt zu erwähnen, daß der Einsatz von Tensiden bei dem erfindungsgemäßen Verfahren nicht mehr unbedingt erforderlich ist. Vermutlich übernehmen die im Rohenzym vorhandenen Polysaccharide teilweise die Aufgabe der Schäumwirkung der Tenside. Diese lagern sich nämlich bei konventionellen Flotationsprozessen als amphotere Stoffe mit ihren hydrophoben Molekülen an die ursprünglich hydrophoben Druckfarbenteilchen an. Durch die Reaktion mit den Härtebildnern des Wassers ist eine Anlagerung an die vorhandenen Luftblasen möglich. Die nicht durch die wasserhärtebildenden Ionen gefällten Seifenbestandteile werden als Schäumer und Dispergiermittel aktiv.

Im folgenden wird die Erfindung unter Bezugnahme auf ein Beispiel näher erläutert:
12 g atro Stoff (Altpapier/Tageszeitungen) werden in etwa 2 cm x 2 cm große Stücke zerrissen. Daraufhin werden 300ml Wasser mit einer ungefähren Calciumhärte von 2,5 mmol zugegeben und bei ca. 40^{o}C per Hand homogenisiert und daraufhin bei 3000 rpm 5 Minuten im Desintegrator desintegriert. Anschließend wird bei einer Stoffdichte von 3,5 % 2 Minuten bei 3000 rpm erneut desintegriert. Anschließend wird auf 1,5 l mit Wasser von 40^{°}C aufgefüllt und der Stoff in eine 1,5 l Flotationszelle gegeben.
Es werden 0,1 - 1,5 % H₂O₂ auf atro Papier bezogen zugegeben. Ebenso werden ca. 2 x 10⁻⁵ % - 2 x 10⁻³ % VA auf atro Papier bezogen zugegeben. Nach Zugabe von 500 bis 5000 IU lignolytischen Enzymen (1 IU = Umsatz von 1 nmol VA/min. in Veratrylaldehyd) wird der Deink-Flotationsprozeß durch gleichzeitige Zugabe von 60 l Luft/Std. und 1200 rpm Rührerdrehzahl in Gang gesetzt. Gleichzeitig wird über eine eingetauchte Redoxelektrode über eine Pumpensteuerung die Dosierung von H₂O₂ und Natrium-Bisulfit-Lösung so dosiert, daß ein mittleres Redoxpotential von ca. 400 mV eingestellt bleibt. Der Prozeß wird 10 - 15 Minuten bei 40^{°}C fortgeführt und der Schmutzstoff mittels eines Schabers abgeschöpft.

## Patentansprüche

1. Verfahren zur Altpapieraufbereitung mit enzymatischer Druckfarbenentfernung durch Flotation
**dadurch gekennzeichnet, daß**
a) unter gleichzeitiger Zudosierung von Oxidations- und Reduktionsmitteln zu einer Altpapier enthaltenden Lösung ein Redoxpotential im Bereich von 200 bis 500 mV eingestellt,
b) durch Zugabe von lignotytischen Enzymen eine ligninabbauende Reaktion gestartet und
c) die Reaktion unter Zugabe von 60 bis 80 l/Std. Luft pro 1,5 l Reaktionsvolumen bei 40°C, o,8 bis 2,5 % Stoffdichte in einer handelsüblichen Flotationsanlage für ca. 10 Minuten fortgeführt wird.

2. Verfahren nach Anspruch 1,
**dadurch gekennzeichnet, daß** nach Schritt c) die Reaktion unter verminderter Luftzufuhr 60 bis 90 Minuten weiterläuft.

3. Verfahren nach Anspruch 1
**dadurch gekennzeichnet, daß** vor Schritt c) die Reaktion unter verminderter Luftzufuhr 60 bis 90 Minuten läuft.

4. Verfahren nach einem der Ansprüche 1 bis 3,
**dadurch gekennzeichnet, daß** das Redoxpotential im Bereich von 250 bis 450 mV liegt.

5. Verfahren nach einem der Ansprüche 1 bis 4,
**dadurch gekennzeichnet,** daß als Oxidationsmittel H₂O₂, O₂ oder Ozon eingesetzt wird.

6. Verfahren nach einem der Ansprüche 1 bis 5,
**dadurch gekennzeichnet,** daß als Reduktionsmittel Ascorbinsäure, Dithionit oder Natrium-Bisulfit eingesetzt werden.

7. Verfahren nach Anspruch 6,
**dadurch gekennzeichnet,** daß Kupfer(II)sulfat auch Mn(II)sulfat, Mn(III)acetat, Fe(II)sulfat, Ti(III)chlorid, Ce(III)nitrat und/oder Ce(IV) amoniumnitrat der Reaktionslösung zugesetzt werden.

8. Verfahren nach Anspruch 7,
**dadurch gekennzeichnet,** daß Zink. Antimon- und Bleisalze der Reaktionslösung zugesatzt werden.

9. Verfahren nach einem der Ansprüche 1 bis 8,
**dadurch gekennzeichnet,** daß phenolische Verbindungen der Reaktionslösung zugesetzt werden.

10. Verfahren nach Anspruch 9,
**dadurch gekennzeichnet,** daß als phenolische Verbindung Veratrylalkohol verwendet wird.

11. Verfahren nach einem der Ansprüche 1 bis 10,
**dadurch gekennzeichnet,** daß als lignolytische Enzyme Phenoloxidasen, Lacrasen und Peroxidasen eingesetzt werden.

12. Verfahren nach einem der Ansprüche 1 bis 11,
**dadurch gekennzeichnet,** daß zusätzlich zu den lignolytischen Enzymen Pektinasen und/oder Hemicellulasen eingesetzt werden.

13. Verfahren nach einein der Ansprüche 1 bis 12,
**dadurch gekennzeichnet,** daß nur die prosthetischen Gruppen der lignolytischen Enzyme eingesetzt werden.

14. Verfahren nach einem der Ansprüche 1 bis 13,
**dadurch gekennzeichnet,** daß der pH-Wert zwischen 2 und 5 liegt.

15. Verfahren nach einem der Ansprüche 1 bis 14,
**dadurch gekennzeichnet,** daß pH-Wert 3 beträgt.

16. Verfahren nach einem der Ansprüche 1 bis 15,
**dadurch gekennzeichnet,** daß Natriumhypochlorit der Reaktionslösung zugesetzt wird.

17. Verfahren nach einem der Ansprüche 1 bis 16,
**dadurch gekennzeichnet,** daß der Reaktionslösung Komplexbildner zugesetzt werden.

18. Verfahren nach Anspruch 17,
**dadurch gekennzeichnet,** daß als Komplexbildner Ehylendiamin-tetraessigsäure (EDTA) oder Diethylentriamin-pentaessigsäure (DTPA) zugesetzt werden.

19. Verfahren nach einem der Ansprüche 1 bis 18,
**dadurch gekennzeichnet,** daß der Reaktionslösung Polysaccharide zugesetzt werden.

20. Verfahren nach Anspruch 19,
**dadurch gekennzeichnet,** daß als Polysaccharide Glukane oder Xanthan eingesetzt werden.

21. Verfahren nach einem der Ansprüche 1 bis 20,
**dadurch gekennzeichnet,** daß der Reaktionslösung Detergenzien zugesetzt werden.

22. Verfahren nach einem der Ansprüche 1 bis 21,
**dadurch gekennzeichnet,** daß der Reaktionslösung Tenside zugesetzt werden.

23. Verfahren nach einem der Ansprüche 1 bis 22,
**dadurch gekennzeichnet,** daß Fettsäuren der Reaktionslösung zugesetzt werden.

24. Verfahren nach Anspruch 23,
**dadurch gekennzeichnet,** daß als Fettsäure Ölsäure eingesetzt wird.

25. Verfahren nach einem der Ansprüche 1 bis 24,
**dadurch gekennzeichnet,** daß Hämverbindungen der Reaktionslösung zugesetzt werden.

26. Verfahren nach Anspruch 25,
**dadurch gekennzeichnet,** daß als Hämverbindung Hämoglobin eingesetzt wird.

27. Verfahren nach einem der Ansprüche 1 bis 26,
**dadurch gekennzeichnet,** daß Perborat der Reaktionslösung zugesetzt wird.

## Claims

1. A process for waste paper recycling with enzymatical printing ink removal by flotation,
characterised in that
a) a redox potential in the range of 200 to 500 mV is adjusted while metered addition of oxidants and reductives to a solution containing waste paper takes place simultaneously,
b) a lignine-decomposing reaction is started by adding lignolytical enzymes and
c) the reaction is continued in a commercial flotation unit for approximately 10 minutes at 40°C, 0.8 to 2.5% consistency during which period 60 to 80 l of air/h per 1.5 l reaction volume is added.

2. A process according to claim 1,
characterised in that the reaction is continued for 60 to 90 minutes after step c), during which time the addition of air is reduced.

3. A process according to claim 1,
characterised in that the reaction runs for 60 to 90 minutes before step c), during which time the addition of air is reduced.

4. A process according to any of the claims 1 to 3,
characterised in that the redox potential is in the range of 250 to 450 mV.

5. A process according to any of the claims 1 to 4,
characterised in that H₂0₂ , O₂ or ozone is used as oxidant.

6. A process according to any of the claims 1 to 5,
characterised in that ascorbic acid, dithionite or sodium bisulphite is used as reductive.

7. A process according to claim 6,
characterised in that cupric sulphate, Mn(II)sulphate, Mn(III)acetate, Fe(II)sulphate, Ti(III)chloride, Ce(III)nitrate and/or Ce( IV)ammonium nitrate are added to the reacting solution.

8. A process according to claim 7,
characterised in that zinc salts, antimonial salts and lead salts are added to the reacting solution.

9. A process according to any of the claims 1 to 8,
characterised in that phenolic compounds are added to the reacting solution.

10. A process according to claim 9 ,
characterised in that veratryl alcohol is used as the phenolic compound.

11. A process according to any of the claims 1 to 10 ,
characterised in that phenoloxidases , laccases and peroxidases are used as lignolytical enzymes.

12. A process according to any of the claims 1 to 11,
characterised in that pectinases and/or hemicellulases are used in addition to the lignolytical enzymes.

13. A process according to any of the claims 1 to 12,
characterised in that only the prosthetic groups of the lignolytical enzymes are used.

14. A process according to any of the claims 1 to 13 ,
characterised in that the pH value is between 2 and 5.

15. A process according to any of the claims 1 to 14 ,
characterised in that the pH value is 3.

16. A process according to any of the claims 1 to 15 ,
characterised in that sodium hypochlorite is added to the reacting solution.

17. A process according to any of the claims 1 to 16 ,
characterised in that complexing agents are added to the reacting solution.

18. A process according to claim 17,
characterised in that ethylene diamine tetraacetic acid (EDTA) or diethylene triamine pentaacetic acid (DTPA) is used as complexing agent.

19. A process according to any of the claims 1 to 18 ,
characterised in that polysaccharides are added to the reacting solution.

20. A process according to claim 19 ,
characterised in that glucanes or xanthane are used as polysaccharides.

21. A process according to any of the claims 1 to 20 ,
characterised in that detergents are added to the reacting solution.

22. A process according to any of the claims 1 to 21,
characterised in that surface-active agents are added to the reacting solution.

23. A process according to any of the claims 1 to 22 ,
characterised in that fatty acids are added to the reacting solution.

24. A process according to claim 23,
characterised in that oleic acid is used as fatty acid .

25. A process according to any of the claims 1 to 24, characterised in that haeme compounds are added to the reacting solution .

26. A process according to claim 25 ,
characterised in that haemoglobin is used as haeme compound.

27. A process according to any of the claims 1 to 26,
characterised in that perborate is added to the reacting solution.

## Revendications

1. Procédé de préparation pour de vieux papiers avec une élimination enzymatique de la couleur d'imprimerie par flottation, caractérisé par le fait
a) qu'un potentiel redox est ajusté dans la gamme allant de 200 à 500 mV tout en additionnant des agents d'oxydation et de réduction à me solution contenant de vieux papiers,
b) que par l'addition d'enzymes lignolitiques est déclenchée une réaction décomposant la lignine et
c) que la réaction est continuée dans un dispositif de flottation d'usage pour une durée d'à peu près 10 minutes, à une température de 40° C et une consistance de 0,8 à 2,5 % tout en ajoutant une quantité d'air de 60 à 80 l/h par 1,5 volume de réaction.

2. Procédé selon la revendication 1, caractérisé par le fait que la réaction est continuée après le pas c) pour me durée de 60 à 90 minutes, l'admission d'air étant réduite.

3. Procédé selon la revendication 1, caractérisé par le fait que la réaction se produit avant le pas c) pour me durée de 60 à 90 minutes, l'admission d'air étant réduite.

4. Procédé selon l'une des revendications 1 à 3, caractérisé par le fait que le potentiel redox est compris entre 250 est 450 mV.

5. Procédé selon l'une des revendications 1 à 4, caractérisé par le fait que l'agent d'oxydation utilisé est de H₂O₂, de O₂ ou de l'ozone.

6. Procédé selon l'une des revendications 1 à 5, caractérisé par le fait que l'agent de réduction utilisé est de l'acide ascorbique, du dithionite ou du bisulfite de sodium.

7. Procédé selon la revendication 6, caractérisé par le fait que l'on ajoute à la solution réactionnelle du sulfate cuivrique mais également du sulfate manganeux, de l'acétate manganique, du sulfate ferreux, du chlorure titaneux, du nitrate céreux et / ou de l'azotate d'ammoniaque cérique.

8. Procédé selon la revendication 7, caractérisé par le fait que l'on ajoute à la solution réactionnelle du zinc ainsi que des sels d'antimoine et de plomb.

9. Procédé selon l'se des revendications 1 à 8, caractérisé par le fait que l'on additionne à la solution réactionnelle des combinaisons phénoliques.

10. Procédé selon la revendication 9, caractérisé par le fait que la combinaison phénolique utilisée est de l'alcool de vératryle.

11. Procédé selon l'une des revendications 1 à 10, caractérisé par le fait que les enzymes lignolitiques utilisées sont des oxydases phénoliques, des laccases ainsi que des peroxydases.

12. Procédé selon l'une des revendications 1 à 11, caractérisé par le fait que l'on utilise outre les enzymes lignolitiques en sus des pectinases et / ou des hémicellulases.

13. Procédé selon l'une des revendications 1 à 12, caractérisé par le fait que l'on n'utilise que les groupes prostétiques des enzymes lignotitiques.

14. Procédé selon l'se des revendications 1 à 13, caractérisé par le fait que la valeur pH est comprise entre 2 et 5.

15. Procédé selon l'me des revendications 1 à 14, caractérisé par le fait que la valeur pH est de 3.

16. Procédé selon l'se des revendications 1 à 15, caractérisé par le fait que l'on ajoute à la solution réactionnelle du hypochlorite de sodium.

17. Procédé selon l'une des revendications 1 à 16, caractérisé par le fait que l'on ajoute à la solution réactionnelle un agent complexant.

18. Procédé selon la revendication 17, caractérisé par le fait que l'agent complexant ajouté est du éthylènediamine-tetraacide acétique (EDTA) ou bien du diéthylènetriamine - pentaacide acétique (DTPA).

19. Procédé selon l'une des revendications 1 à 18, caractérisé par le fait que l'on additionne à la solution réactionnelle des polysaccharides.

20. Procédé selon la revendication 19, caractérisé par le fait que les polysaccharides utilisés sont des glucanes ou du xanthane.

21. Procédé selon l'une des revendications 1 à 20, caractérisé par le fait que l'on ajoute à la solution réactionnelle des détergents.

22. Procédé selon l'une des revendications 1 à 21, caractérisé par le fait que l'on ajoute à la solution réactionnelle des agents de surface.

23. Procédé selon l'me des revendications 1 à 22, caractérisé par le fait que l'on additionne à la solution réactionnelle des acides gras.

24. Procédé selon la revendication 23, caractérisé par le fait que l'acide gras utilisé est de l'acide oléique.

25. Procédé selon l'une des revendications 1 à 24, caractérisé par le fait que l'on ajoute à la solution réactionnelle des combinaisons hèmes.

26. Procédé selon la revendication 25, caractérisé par le fait que l'on utilise en tant que combinaison hème de la hémoglobine.

27. Procédé selon l'une des revendications 1 à 26, caractérisé par le fait que l'on ajoute à la solution réactionnelle du perborate.
